# EUROPEAN PATENT APPLICATION

(11) **EP 1 475 121 A1**
(43) Date of publication of application: **10.11.2004**
(21) Application number: 04011115.5
(22) Date of filing: 10.05.2004
(51) Int. Cl.: A61M 25/00

(54) **Laminated catheter comprising ultra high molecular weight high density polyethylene**

(30) Priority: 09.05.2003 US 434774
(71) Applicant: Medtronic Vascular, Inc., Santa Rosa, CA 95403 (US)
(72) Inventor: Horrigan, John, Beverly Massachusetts 01915 (US)
(74) Representative: Hughes, Andrea Michelle

(57) **Abstract**

A tubular medical catheter 10 having an inner liner 30 comprising ultra high molecular weight high density polyethylene, and methods of making the same, are provided herein.

## Description

### FIELD OF THE INVENTION

The present invention relates to catheters having an inner liner comprising an ultra high molecular weight high density polyethylene (UHMW-HD PE). More specifically, the invention relates to catheters having an inner liner comprising an ultra high molecular weight high density polyethylene, the surface of which has been modified by a laser to enhance bonding between the inner liner and a polymer outer shell, and methods for making the same.

### BACKGROUND OF THE INVENTION

A number of intravascular procedures are currently utilized to treat a stenosis within a body vessel of a human being. A common intravascular procedure is referred to as percutaneous transluminal coronary angioplasty (PTCA or hereinafter "angioplasty"). During a typical angioplasty procedure, a guidewire is initially positioned within the body vessel and a guiding catheter is positioned over the guidewire. Next, a balloon catheter having an inflatable balloon is advanced through the guiding catheter and vessel until the balloon is adjacent to the stenosis. Subsequently, inflation of the balloon compresses the stenosis and dilates the body vessel.

During many diagnostic or interventional catheterization procedures, it is necessary to route the catheter from an entry point, such as the femoral artery, to a target location within the vasculature. When a guiding catheter is properly placed into position, the balloon catheter, for example, should be able to be turned, pulled, and pushed so that the distal end of the catheter can navigate the twists and turns of the guiding catheter on its path to the final location. In this regard, a guiding catheter having an inner liner that is manufactured using a lubricious material is helpful. Traditionally, Teflon® materials such as perfluoro ethylene-propylene (FEP) or polytetrafluoroethylene (PTFE) have been used to manufacture inner liners.

The lubricious nature of Teflon® materials, however, makes them more difficult to bond to, for example, an outer shell without additional treatment of the surface of the inner liner. Typically, such surface treatment is carried out by a chemical etch process where, for example, the Teflon® material is subjected to an etch bath for a period of time sufficient to modify the surface of the Teflon® and enhance bonding to, for example, the outer shell.

Another drawback to using inner liners manufactured using Teflon® materials such as PTFE is that these materials are often not melt-extrudable. As a consequence, PTFE, for example, can only be purchased in relatively short lengths, termed "sticks," and thus requires inefficient one-at-a-time catheter manufacturing processes. In contrast, reel-to-reel processes are more cost-efficient than assembling catheters one-at-a-time.

Accordingly, there is a need for a medical catheter that comprises an inner liner manufactured from a lubricious material that can bond to additional polymer materials, and that is simple to manufacture. The present invention addresses these needs, as well as other problems associated with existing medical catheters. The present invention also offers further advantages over the prior art and solves other problems associated therewith.

### SUMMARY OF THE INVENTION

The present invention is directed to medical catheters adapted for use within a body vessel. The medical catheter comprises a tubular catheter shaft having a distal end that fits within the body vessel. The tubular catheter shaft comprises an inner liner and an outer shell. The medical catheter comprises an inner liner that comprises ultra high molecular weight high-density polyethylene - UHMW-HD PE.

In some embodiments, at least one region of the surface of the inner liner has been modified with a laser. The laser modification of the surface of the inner liner etches the surface of the inner liner and enhances bonding of the inner liner to other polymers,. such as those that comprise the outer shell.

In some embodiments, an adhesive bonds the modified surface of the inner liner to the outer shell. An example of a suitable adhesive is an epoxy.

The present invention is also directed to methods for making a medical catheter having an inner liner comprising UHMW-HD PE. A medical catheter comprising a melt-extrudable UHMW-HD PE is provided. The surface of the inner liner is modified in a manner so as to enhance the bonding of the inner liner to another polymer. In some embodiments, the inner liner is modified using a laser to etch the surface of the inner liner. Upon modification of the surface of the inner liner, an adhesive is applied to enhance bonding to another polymer. In some embodiments, the inner liner and outer shell are bonded with adhesive, such as an epoxy.

### BRIEF DESCRIPTION OF THE DRAWINGS

The novel features of this invention, as well as the invention itself, both as to its structure and its operation, will be best understood from the accompanying drawings, taken in conjunction with the accompanying description of preferred embodiments given by way of example only, in which similar reference characters refer to similar parts, and in which:

FIG. 1 is a perspective view, in partial cutaway, of a medical catheter having features of the present invention;

FIG. 2 is an enlarged cutaway view of a portion of the medical catheter of FIG. 1;

FIG. 3 is a perspective illustration of the medical catheter positioned within a patient; and

FIG. 4 is an enlarged side plan assembly view of a portion of the catheter shaft illustrating a groove.

### DETAILED DESCRIPTION OF THE INVENTION

The present invention is directed to a catheter that has an inner liner comprising UHMW-HD PE. Any multi-layered medical catheter can be modified to have an inner liner comprising UHMW-HD PE. The catheters described herein are merely exemplary and the invention should not be construed to be limited to only the catheters described herein.

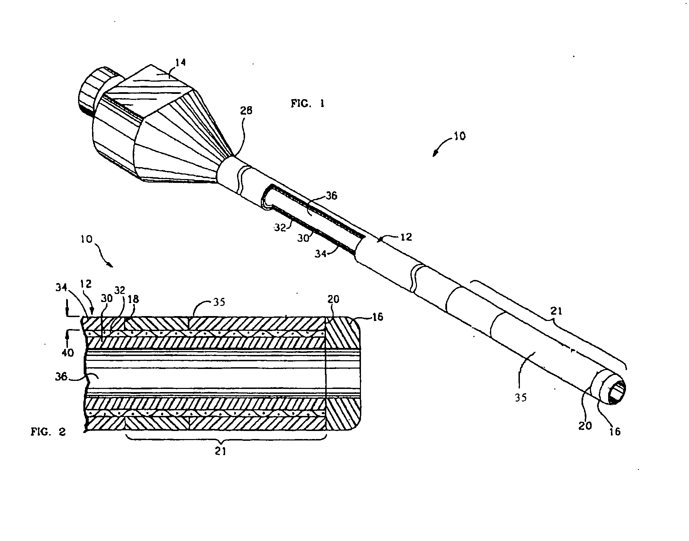

Referring to FIGS. 1, 2, and 4, a first embodiment of medical catheter 10 having features of the present invention includes tubular catheter shaft 12, hub 14, and tubular flexible tip 16. Catheter shaft 12 can optionally include groove 18, which is cut out of catheter shaft 12 near distal end 20 of catheter shaft 12.

Medical catheter 10 illustrated herein is utilized to guide an interventional catheter (not shown) and is commonly referred to as a guiding catheter. FIG. 3 illustrates a portion of medical catheter 10 and guidewire 22 positioned in body vessel 24 of patient 26 during a procedure. The location of entry into patient 26 and the location of distal end 20 in patient 26 are merely exemplary.

Referring back to FIGS. 1 and 2, hub 14 is secured to proximal end 28 of catheter shaft 12 while flexible tip 16 is secured to distal end 20 of catheter shaft 12. The physician manipulates hub 14 and proximal end 28 to position medical catheter 10 in body vessel 24. Flexible tip 16 assists in guiding medical catheter 10 in body vessel 24 and minimizes the trauma to vessel 24 and coronary ostium (not shown).

Flexible tip 16 is made of a relatively soft material when compared to the catheter shaft 12. Suitable materials for flexible tip 16 may include polymers such as a polyether block amide ("PEBA") having a hardness of about 40 Shore D. Depending upon the materials utilized, hub 14 and flexible tip 16 can be thermally bonded or attached with an adhesive (not shown) to catheter shaft 12. Those skilled in the art will recognize alternate ways to attach hub 14 and flexible tip 16 and that alternate materials can be utilized for flexible tip 16.

In the embodiment illustrated in FIGS. 1 and 2, tubular catheter shaft 12 includes inner liner 30, optional reinforcing section 32, and outer shell 34. Further, when the catheter comprises groove 18, fill section 35 may be positioned in groove 18. Inner liner 30 is tubular and defines lumen 36, which is sized and shaped to receive, for example, guidewire 22 and subsequently an interventional catheter (not shown).

Typically, inner liner 30 is manufactured by extruding UHMW-HD PE, which provides good flexibility and movement over guidewire 22. The composition of the inner liner, however, is not limited to only this polymer and any suitable polymer having lubricious properties and which is melt-extrudable can be used so that the catheter has the desired properties. A suitable inner liner 30 has an inner diameter of between about 0.08 and 0.09 inches (2.032 - 2.286 mm) and an inner liner thickness of about 1.5 mils (0.0381 mm). An additional lubricious coating (not shown) may be added to lumen 36 of inner liner 30 to facilitate even more movement of inner liner 30 over guidewire 22 and the interventional catheter within lumen 36.

Outer shell 34 provides support to catheter shaft 12 and covers reinforcing section 32 to protect body vessel 24 from reinforcing section 32. Further, outer shell 34 prevents reinforcing section 32 from unwrapping. Outer shell 34 is tubular and coaxial with inner liner 30 and optional reinforcing section 32. A suitable outer shell 34 has an inner diameter of about 0.1 inches ( 2.54 mm) and wall thickness 40 of about 2.5 mils (0.0635 mm).

Typically, outer shell 34 is manufactured by extruding a polymer over the reinforcing section 32. A suitable shell material for outer shell 34 is a nylon sold under the trademark "TROGAMID" by Creanova (Somerset, N.J.). The shell material may have a hardness of approximately 81 Shore D. Additionally, a lubricious coating (not shown) may be added to outer shell 34 to facilitate movement of catheter shaft 12 within vessel 24.

Those skilled in the art will recognize alternative ways to manufacture inner liner 30, reinforcing section 32, and outer shell 34 and that alternate materials can be utilized for inner liner 30, reinforcing section 32, and outer shell 34. Those skilled in the art will also recognize alternate ways to apply reinforcing section 32 on inner liner 30.

The surface of inner liner 30 is modified so as to enhance bonding with outer shell 34. UHMW-HD PE, however, is chemically resistant and, thus, cannot be chemically etched so as to create a surface that can bond to outer shell 34. A solution to this problem, however, is to utilize a laser to modify the surface of the UHMW-HD PE inner liner 30 to enhance bonding between UHMW-HD PE and outer shell 34. After the inner liner surface has been prepared, an adhesive, such as an epoxy, can be used to bond inner liner 30 to outer shell 34.

Outer shell 34 is extruded or molded over inner layer 30. Next, the composite catheter tubing can be severed at desired cut points, forming multiple sub-assemblies of intended catheter length. Finally, catheters 10 are finished by securing the remaining components.

In some embodiments, inner liner 30 and/or outer shell 34 comprise unfilled or low-loaded thermoplastic polymers. For example, inner liner 30 and outer shell 34 each, independently, may include a radiopaque material and/or filler and/or colorant, such that the total content of the radiopaque material and/or filler and/or colorant in inner liner 30 and/or outer shell 34 is between about 0.1% and about 10%, or between about 0.1% and about 5%, or between about 0.1% and about 2% of the total weight making up inner liner 30 and/or outer shell 34. In some embodiments, inner liner 30 and outer shell 34 each, independently, may exclude a radiopaque material and/or filler and/or colorant, thus having 0% by weight of the total weight making up inner liner 30 and/or outer shell 34. An unfilled inner liner 30 and/or outer shell 34 have the advantages of retaining mechanical integrity and modulus of elasticity.

In some embodiments, an optional reinforcing section 32 enhances the torsional strength and prevents or reduces kinking of catheter shaft 12 during movement of medical catheter 10 in body vessel 24. Reinforcing section 32 can be embedded between inner liner 30 and outer shell 34 and is substantially coaxial with inner liner 30 and outer shell 34. Reinforcing section 32 may be formed by braiding wire mesh around inner liner 30. Subsequently, outer shell 34 is formed around reinforcing section 32 by applying materials making up the outer shell. In any of the above embodiments of the invention, the optional reinforcing section 32 can be created around etched inner liner 30 before outer shell 34 is formed.

There are many benefits of a catheter having an inner liner comprising UHMW-HD PE. For example, such a catheter possesses a lubricious inner liner, which provides enhanced guidance properties. Such a catheter also can be made in a reel-to-reel fashion, which provides an increased ease of manufacture compared to catheters having PTFE inner liners.

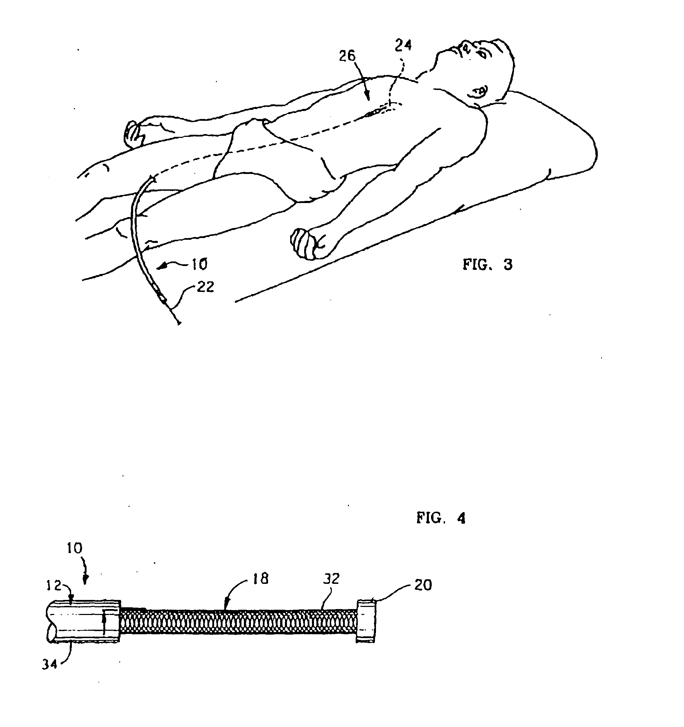

As illustrated in FIG. 4, catheter shaft 12 can optionally include groove 18, which is cut out of catheter shaft 12 near distal end 20 of catheter shaft 12, as described in U.S. Patent No. 6,059,769. Groove 18 provides flexibility at distal end 20 of catheter shaft 12 without compromising the durability and torsional strength of catheter shaft 12. Further, groove 18 functions as transitional region 21 between relatively stiff catheter shaft 12 and flexible tip 16. This prevents or reduces kinking and/or collapsing of medical catheter 10. As a result thereof, medical catheter 10 has improved tracking and movement in the vessel. Fill section 35 may be positioned in groove 18. An embodiment of the invention may combine filled groove 18 with one or more regions of selectively varied lamination 55 to achieve a desirable combination of mechanical properties.

While the particular medical catheter 10 as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A medical catheter (10) adapted for use within a body vessel, the medical catheter comprising:
a tubular catheter shaft (12) comprising an inner liner (30) and outer shell (34) and having a distal end which fits within the body vessel;
wherein the inner liner comprises ultra high molecular weight high density polyethylene.

2. The medical catheter of claim 1 wherein the surface of the inner liner has been modified by a laser.

3. The medical catheter of claim 2 wherein an adhesive bonds the modified surface of the inner liner to the outer shell.

4. The medical catheter of claim 3 wherein the adhesive is an epoxy.

5. The medical catheter of any preceding claim wherein the outer shell (34) comprises nylon or nylon 12.

6. The medical catheter of any preceding claim wherein the inner liner (30) is unfilled or low-loaded with filler, radiopaque material, or colorant.

7. The medical catheter of any preceding claim wherein the outer shell (34) is unfilled or low-loaded with filler, radiopaque material, or colorant.

8. The medical catheter of any preceding claim further comprising a reinforcing section (32) positioned between the inner liner (30) and the outer shell (34).

9. The medical catheter of claim 8 wherein the reinforcing section is a braid.

10. A method for making a medical catheter (10) comprising the steps of:
providing a medical catheter having an inner liner (30) comprising a melt-extrudable ultra high molecular weight high density polyethylene;
modifying the surface of the inner liner;
applying an adhesive to the modified surface of the inner liner; and
applying material to form an outer shell over the adhesive on the modified surface of the inner liner.

11. The method of claim 10 wherein the modifying step comprises laser etching.

12. The method of claim 11 wherein the adhesive is an epoxy.

13. The method of claim 10, 11 or 12 further comprising applying a reinforcing section (32) over the inner liner prior to applying material to form the outer shell.

14. The method of claim 13 wherein the reinforcing section is a braid.
